(19) Europäisches Patentamt
European Patent Office
Office européen des brevets

(11) Veröffentlichungsnummer: **0 018 493 B1**

(12) # EUROPÄISCHE PATENTSCHRIFT

(45) Veröffentlichungstag der Patentschrift: **01.12.82**

(51) Int. Cl.³: **C 07 D 241/44**, A 61 K 31/495

(21) Anmeldenummer: **80101525.6**

(22) Anmeldetag: **22.03.80**

(54) **2-Sulfonyl-chinoxaline, Verfahren zu ihrer Herstellung sowie ihre Verwendung als Mikrobizide.**

(30) Priorität: **05.04.79 DE 2913728**

(43) Veröffentlichungstag der Anmeldung: **12.11.80 Patentblatt 80/23**

(45) Bekanntmachung des Hinweises auf die Patenterteilung: **01.12.82 Patentblatt 82/48**

(84) Benannte Vertragsstaaten: **AT BE CH DE FR GB IT NL SE**

(56) Entgegenhaltungen:

**DE-A-2 312 730**
**GB-A-1 293 850**

**Chemical Abstracts Band 49, Nr. 12, 25. Juni 1955, Columbus, Ohio, USA F.J. WOLF et al. «2-Quinoxalinethiol and derivatives»**

**Die Akte enthält technische Angaben, die nach dem Eingang der Anmeldung eingereicht wurden und die nicht in dieser Patentschrift enthalten sind.**

(73) Patentinhaber: **BAYER AG, Zentralbereich Patente, Marken und Lizenzen, D-5090 Leverkusen 1, Bayerwerk (DE)**

(72) Erfinder: **Sasse, Klaus, Dr., Pützweg 13, D-5060 Bergisch-Gladbach 2 (DE)**
Erfinder: **Haller, Ingo, Dr., Viktoriastrasse 99, D-5600 Wuppertal-1 (DE)**
Erfinder: **Plempel, Manfred, Dr., Pahlkestrasse 5, D-5600 Wuppertal-1 (DE)**
Erfinder: **Zeiler, Hans-Joachim, Dr., Windrather Strasse 188, D-5620 Velbert 15 (DE)**
Erfinder: **Metzger, Karl Georg, Dr., Pahlkestrasse 15, D-5600 Wuppertal-1 (DE)**
Erfinder: **Haberkorn, Axel, Dr., Fuhlrottstrasse 99, D-5600 Wuppertal-1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 018 493 B1

# 2-Sulfonyl-chinoxaline, Verfahren zu ihrer Herstellung sowie ihre Verwendung als Mikrobizide

Die Erfindung betrifft neue 2-Sulfonyl-chinoxaline, Verfahren zu ihrer Herstellung sowie ihre Verwendung als Mittel gegen parasitäre Mikroorganismen.

Es ist bereits bekannt geworden, dass in 2-Stellung sulfonylsubstituierte Chinoxalin-4-oxide und 1,4-Dioxide Wirkung gegen phytopathogene Krankheitserreger aufweisen. Von entsprechenden Chinoxalinen ohne Sauerstoffsubstitution in 1- oder 1,4-Stellung wird dies nur der im Phenylenrest unsubstituierten Methylsulfonylverbindung unterstellt (vgl. DOS 2 312 730). Verbindungen dieser Art besitzen keine wesentlichen Hemmaktivitäten gegenüber humanpathogenen Pilzen und Bakterien sowie gegenüber Schimmelpilzen.

Es wurde gefunden, dass die neuen 2-Sulfonyl-chinoxaline der Formel I mit mindestens einem Substituenten der genannten Art im ankondensierten Phenylenrest, hohe Wirksamkeit gegenüber humanpathogenen Pilzen und Bakterien sowie Schimmelpilzen und Trichomonaden entfalten. Sie können daher als Arzneimittel für Menschen und Tiere verwendet werden.

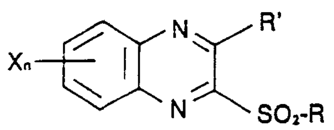

(I)

In Formel I bedeuten:

X ein Halogenatom, Nitro oder Trifluormethyl,

R' ein Wasserstoffatom, einen Alkylrest mit 1 bis 4 Kohlenstoffatomen, einen Cycloalkylrest mit 5 oder 6 Kohlenstoffatomen oder einen Phenylrest, der weiterhin substituiert sein kann durch Halogen, Nitro oder Trifluormethyl,

R einen Alkylrest mit 1 bis 6 Kohlenstoffatomen, der durch Halogen, einen Alkoxyrest mit bis zu 4 C-Atomen, eine Amino- oder Dimethylaminogruppe substituiert sein kann, einen Cycloalkylrest mit 5 oder 6 Kohlenstoffatomen, einen Phenylalkylrest mit 1 bis 3 Kohlenstoffatomen im Alkylteil, dessen Phenylrest weiterhin durch Halogen, Nitro, Methyl, Methoxy oder Trifluormethyl substituiert sein kann oder einen Phenylrest, der durch Alkyl mit 1-4 C-Atomen, Trifluormethyl, Alkoxy mit 1-4 C-Atomen oder Halogen substituiert sein kann, und

n 1-4.

Weiterhin wurde gefunden, dass man 2-Sulfonylchinoxaline der Formel I erhält, wenn man 2-Sulfenyl-chinoxaline der Formel II, in welcher X, R, R' und n die oben genannte Bedeutung besitzen, mit Oxidationsmitteln umsetzt.

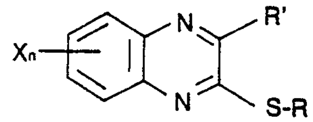

(II)

Die erfindungsgemässen 2-Sulfonyl-chinoxaline zeigen eine erheblich höhere antimykotische und antibakterielle Wirkung als die aus dem Stand der Technik bekannten verwandten Verbindungen ohne Kernsubstitution bzw. mit N-Oxid-Gruppen. Diese neuen Stoffe stellen somit eine Bereicherung der Technik dar.

Die erfindungsgemässen neuen Verbindungen der Formel I werden durch Einwirkung von Oxidationsmitteln auf entsprechend substituierte 2-Sulfenylchinoxaline der Formel II hergestellt.

Am Beispiel der Oxidation des 6-Chlor-2-methylmercapto-chinoxalins zum 6-Chlor-2-methylsulfonyl-chinoxalin sei das Herstellungsverfahren formelmässig näher erläutert:

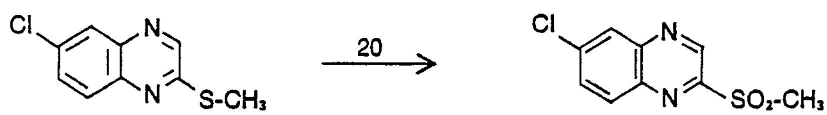

Die Ausgangsstoffe sind durch die Formel II allgemein definiert. In der Formel II haben X, R, R' und n die oben angegebenen Bedeutungen.

Für die Herstellung der Verbindungen I aus den Verbindungen II kommen mehrere, z. T. für ähnliche Reaktionen bereits in der Literatur bekannte Oxidationsmittel in Frage, z. B. peroxidische Verbindungen, Halogene oder Metallverbindungen polyvalenter Metalle in höheren Wertigkeitsstufen. Vorzugsweise werden folgende Oxidationsmittel verwendet:

a) Wasserstoffperoxid. Es werden wenigstens zwei Mol pro Mol der Verbindung II benötigt. Vorzugsweise arbeitet man mit einem Überschuss von 0,1 bis 0,5 Mol. Als Lösungsmittel können verschiedene inerte organische Solventien eingesetzt werden. Vorzugsweise arbeitet man in Essigsäure oder deren Mischungen mit Wasser. Die Reaktionstemperaturen betragen 20-120°C, vorzugsweise 50-90°C (vgl. J. chem. Soc. 1957, 3236).

b) Elementares Chlor in Gegenwart von Wasser.

Es werden wenigstens zwei Mol $Cl_2$ pro Mol der Verbindung II benötigt. Man kann in reinem Wasser arbeiten. Einen gleichmässigeren Reaktionsverlauf erreicht man durch die Verwendung von Lösungsvermittlern wie Alkohole, z.B. Methanol oder Glykolmonomethyläther oder niedere Carbonsäuren, z. B. Ameisen-, Essig- oder Propionsäure. Das Mischungsverhältnis Wasser : Lösungsvermittler kann zwischen 10:1 und 1:10 variiert werden. Die Reaktionstemperatur sollte +10°C nicht überschreiten. Vorzugsweise arbeitet man bei 0-5°C.

c) Permanganat. Für die vollständige Umsetzung werden 4/3 Mol pro Mol der Verbindung II benötigt, vorzugsweise arbeitet man mit einem Überschuss des Oxidationsmittels von 0,2 bis 0,5 Mol. Als Verdünnungsmittel dient Wasser oder Mischungen aus diesem mit mischbaren organischen Lösungsmitteln, z. B. Aceton, Dioxan, Tetrahydrofuran oder niedere Carbonsäuren wie Ameisen-, Essig- und Propionsäure. Die Reaktionstemperaturen betragen 0-100°C, vorzugsweise 10-70°C.

Die Ausgangsprodukte der Formel II sind grossenteils noch nicht bekannt. Sie lassen sich aber nach grundsätzlich bekannten Methoden herstellen, vorzugsweise

A: durch Einwirkung von Mercaptanen und Thiophenolen III auf 2-Halogen-chinoxaline der Formel IV in Gegenwart säurebindender Mittel:

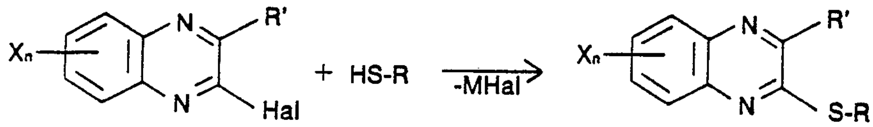


(IV) (III) (II)

In Formel IV haben X, n und R' die oben angegebene Bedeutung. Hal steht für Chlor oder Brom. In III hat R die gleiche Bedeutung wie in I.

Die Umsetzungen werden in inerten organischen Lösungsmitteln wie Kohlenwasserstoffen, Ethern, cyclischen Ethern oder Ketonen durchgeführt oder in deren Mischungen mit Wasser. Als säurebindende Mittel werden vorzugsweise Alkali- und Erdalkali-oxide, -hydroxide oder -carbonate oder tertiäre Amine verwendet. Die Reaktionstemperaturen betragen 0-100°C.

B: durch Einwirkung von Alkylhalogeniden, Alkylsulfonaten oder (Di)alkylsulfaten V auf 2-Mercapto-chinoxaline der Formel VI, wobei in den Formeln V und VI X, n, R und R' die oben angegebene Bedeutung besitzen und Y für Halogen oder $-O-SO_2-R''$ oder $-O-SO_2-O-R$ steht.

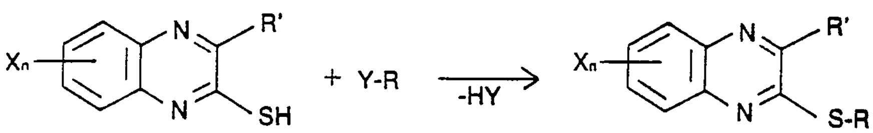


(VI) (V) (II)

Diese Reaktionen werden ebenfalls in inerten organischen Lösungsmitteln, wie Alkohole, Kohlenwasserstoffe, Ether oder Ketone bzw. in deren Mischungen mit Wasser durchgeführt. Als säurebindende Mittel verwendet man die gleichen wie unter A beschrieben. Die Reaktionstemperaturen betragen 0-100°C.

Soweit die Verbindungen VI noch nicht bekannt sind, lassen sie sich nach bekannten Verfahren, z. B. aus den Verbindungen IV durch Einwirkung von Salzen des Schwefelwasserstoffs oder aus entsprechenden 2-Hydroxy-derivaten durch Umsetzung mit Phosphor(V)-sulfid herstellen.

Als neue Wirkstoffe seien im einzelnen genannt:

6-Chlor-2-methylsulfonyl-chinoxalin
6-Chlor-2-äthylsulfonyl-chinoxalin
6-Chlor-2-propylsulfonyl-chinoxalin
6-Chlor-2-isopropylsulfonyl-chinoxalin
6-Chlor-2-butylsulfonyl-chinoxalin
6-Chlor-2-isobutylsulfonyl-chinoxalin
6-Chlor-2-tert.butylsulfonyl-chinoxalin
6-Chlor-2-(2-chlor-äthylsulfonyl)-chinoxalin
6-Chlor-2-(2-äthoxy-äthylsulfonyl)-chinoxalin
6-Chlor-2-(2-amino-äthylsulfonyl)-chinoxalin
6-Chlor-2-(2-dimethyamino-äthylsulfonyl)-chinoxalin
6-Chlor-2-cyclopentylsulfonyl-chinoxalin
6-Chlor-2-cyclohexylsulfonyl-chinoxalin
6-Chlor-2-benzylsulfonyl-chinoxalin
6-Chlor-2-(4-chlor-benzylsulfonyl)-chinoxalin
6-Chlor-2-(3,4-dichlor-benzylsulfonyl)-chinoxalin
6-Chlor-2-(4-methyl-benzylsulfonyl)-chinoxalin
6-Chlor-2-(4-nitro-benzylsulfonyl)-chinoxalin
6-Chlor-2-(4-methoxy-benzylsulfonyl)-chinoxalin
6-Chlor-2-(4-trifluormethyl-benzylsulfonyl)-chinoxalin
6-Chlor-2-(2-phenyl-äthylsulfonyl)-chinoxalin
6-Chlor-2-(3-phenyl-propylsulfonyl)-chinoxalin
6-Chlor-2-phenylsulfonyl-chinoxalin
6-Chlor-2-(4-chlor-phenylsulfonyl)-chinoxalin
6-Chlor-2-(4-brom-phenylsulfonyl)-chinoxalin
6-Chlor-2-(4-tert.butyl-phenylsulfonyl)chinoxalin
6-Chlor-2-(4-trifluormethyl-phenylsulfonyl)-chinoxalin
6-Chlor-2-(4-methoxy-phenylsulfonyl)-chinoxalin
6-Chlor-2-(4-nitro-phenylsulfonyl)-chinoxalin
7-Chlor-2-methylsulfonyl-chinoxalin
7-Chlor-2-benzylsulfonyl-chinoxalin
7-Chlor-2-phenylsulfonyl-chinoxalin
6,7-Dichlor-2-methylsulfonyl-chinoxalin
6,7-Dichlor-2-phenylsulfonyl-chinoxalin
6,8-Dichlor-2-methylsulfonyl-chinoxalin
6,8-Dichlor-2-phenylsulfonyl-chinoxalin
5,6,8-Trichlor-2-methylsulfonyl-chinoxalin
5,6,8-Trichlor-2-phenylsulfonyl-chinoxalin
5,6,7,8-Tetrachlor-2-methylsulfonyl-chinoxalin
5,6,7,8-Tetrachlor-2-phenylsulfonyl-chinoxalin

6-Brom-2-methylsulfonyl-chinoxalin
6-Brom-2-phenylsulfonyl-chinoxalin
6-Trifluormethyl-2-methylsulfonyl-chinoxalin
6-Trifluormethyl-2-phenylsulfonyl-chinoxalin
6-Nitro-2-methylsulfonyl-chinoxalin
6-Nitro-2-phenylsulfonyl-chinoxalin
6-Chlor-3-methyl-2-methylsulfonyl-chinoxalin
6-Chlor-3-methyl-2-phenylsulfonyl-chinoxalin
6,8-Dichlor-3-methyl-2-methylsulfonyl-chinoxalin
6,8-Dichlor-3-methyl-2-phenylsulfonyl-chinoxalin
6-Chlor-3-äthyl-2-methylsulfonyl-chinoxalin
6-Chlor-3-propyl-2-methylsulfonyl-chinoxalin
6-Chlor-3-tert.butyl-2-methylsulfonyl-chinoxalin
6-Chlor-3-cyclohexyl-2-methylsulfonyl-chinoxalin
6-Chlor-3-phenyl-2-methylsulfonyl-chinoxalin
6-Chlor-3-phenyl-2-benzylsulfonyl-chinoxalin
6-Chlor-3-(4-chlor-phenyl)-2-methylsulfonyl-chinoxalin
6-Chlor-3-(3-trifluormethyl-phenyl)-2-methylsulfonyl-chinoxalin
6-Chlor-3-(4-nitro-phenyl)-2-methylsulfonyl-chinoxalin

Die neuen Wirkstoffe weisen hohe Wirkung gegen Bakterien, Pilze und Trichomonaden auf.

Beispiele

Beispiel 1

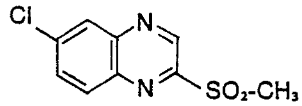

21,1 g (0,1 Mol) 6-Chlor-2-methylmercapto-chinoxalin werden in einer Mischung aus 200 ml Essigsäure und 60 ml Wasser suspendiert. Bei Einhalten einer Temperatur von 0-5°C wird gasförmiges Chlor bis zur Sättigung eingeleitet und dann noch 2 Stunden bei der gleichen Temperatur nachgerührt. Durch Einblasen von Stickstoff wird der Chlorüberschuss entfernt, anschliessend das Reaktionsgemisch mit 1 Liter Wasser verdünnt. Die abgeschiedenen Kristalle werden abgesaugt, getrocknet und aus Waschbenzin umkristallisiert. Man erhält 18,9 g (78% der Theorie) 6-Chlor-2-methylsulfonyl-chinoxalin vom Schmelzpunkt F: 122-124°C.

In entsprechender Weise werden Verbindungen der Formel I

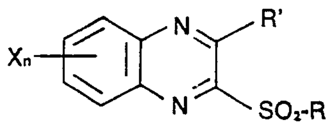

(I)

hergestellt.

| Beispiel Nr. | $X_n$ | R' | R | F (°C) | Umkrist. aus | Ausbeute (%) |
|---|---|---|---|---|---|---|
| 2 | 6-Cl | H | $C_2H_5$ | 132-134 | Äthanol | 82 |
| 3 | 6-Cl | H | $C_3H_7$ | 122-124 | Waschbenzin | 69 |
| 4 | 6-Cl | H | $C_4H_9$ | 99-100 | Waschbenzin | 87 |
| 12 | 6,8-$Cl_2$ | H | $CH_3$ | 143-144 | Tetra | 79 |
| 17 | 6,8-$Cl_2$ | H | 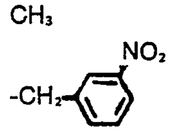 | 154-156 | Essigester/Ligroin | 65 |
| 19 | 6,8-$Cl_2$ | H | $CH_2$-$CH_2$-$N(CH_3)_2$ | 94- 95 | Äthanol | 91 |
| 20 | 6,8-$Cl_2$ | H | 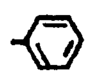 | 149-150 | Waschbenzin | 88 |
| 21 | 6,7-$Cl_2$ | H | $CH_3$ | 149-150 | Toluol | 77 |
| 22 | 6,7-$Cl_2$ | H | $C_2H_5$ | 135-136 | Waschbenzin | 92 |
| 23 | 6,7-$Cl_2$ | H | $C_3H_7$ | 106-108 | Äthanol | 87 |
| 24 | 6,7-$Cl_2$ | H | $C_4H_9$ | 98-100 | Waschbenzin | 85 |
| 31 | 6-$CF_3$ | H | $C_4H_9$ | 85- 86 | Waschbenzin | 67 |
| 33 | 6-$CF_3$ | H | 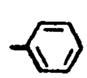 | 125-126 | Waschbenzin | 73 |

Beispiel 5

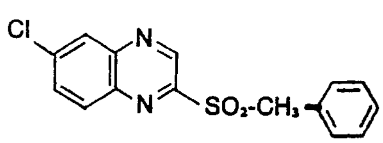

28,7 g 6-Chlor-2-benzylmercapto-chinoxalin werden in 800 ml Essigsäure gelöst. Nach Zugabe von 200 ml Wasser trägt man bei Raumtemperatur portionsweise 31,6 g (0,2 Mol) Kaliumpermanganat ein. Diese Mischung wird 8 Stunden bei Raumtemperatur gerührt und über

Nacht stehen lassen. Dann tropft man soviel einer konzentrierten Natriumhydrogensulfidlösung ein, bis alles überschüssige Permanganat und Mangan(IV)-oxid entfärbt sind. Die mit 1 Liter Wasser verdünnte Mischung wird abgesaugt. Die Kristalle werden getrocknet und aus Toluol umkristallisiert. Man erhält 26 g (81,5% d. Th.) 6-Chlor-2-benzyl-sulfonyl-chinoxalin vom Schmelzpunkt F: 176°C.

In entsprechender Weise werden Verbindungen der Formel I

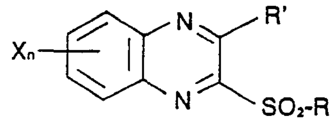

hergestellt.

| Beispiel Nr. | $X_n$ | R' | R | F (°C) | Umkrist. aus | Ausbeute (%) |
|---|---|---|---|---|---|---|
| 6 | 6-Cl | H | 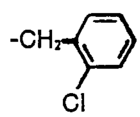 | 181-182 | Toluol | 85 |
| 7 | 6-Cl | H | 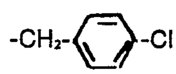 | 196-198 | Toluol | 87 |
| 8 | 6-Cl | H | 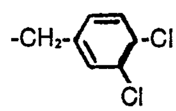 | 200-201 | Toluol | 73 |
| 9 | 6-Cl | H | 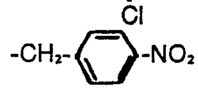 | > 250 | Waschbenzin | 68 |
| 10 | 6-Cl | H | 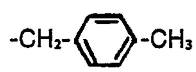 | 193-194 | Toluol | 78 |
| 18 | 6,8-$Cl_2$ | H | 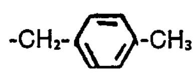 | 142-143 | Toluol | 67 |
| 25 | 5,7-$Cl_2$ | H | 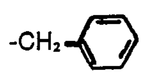 | 174-175 | Waschbenzin | 62 |
| 26 | 5,6,8-$Cl_3$ | H | $CH_3$ | 178-180 | Waschbenzin | 83 |
| 27 | 5,6,8-$Cl_3$ | H | 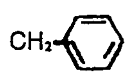 | 158-160 | Waschbenzin | 74 |
| 29 | 6-$NO_2$ | H | 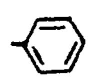 | 200-202 | Toluol | 73 |
| 30 | 6-$CF_3$ | H | $CH_3$ | 128-130 | Waschbenzin | 65 |
| 32 | 6-$CF_3$ | H | 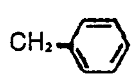 | 144-145 | Waschbenzin | 67 |
| 34 | 6-(7)Cl | $CH_3$ | $CH_3$ | 90- 92 | Waschbenzin | 53 |
| 35 | 6-(7)Cl | $CH_3$ | $C_4H_9$ | 46- 48 | Waschbenzin | 66 |
| 36 | 6-(7)Cl | $CH_3$ | 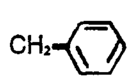 | 132-134 | Waschbenzin | 83 |

| Beispiel Nr. | $X_n$ | R' | R | F (°C) | Umkrist. aus | Ausbeute (%) |
|---|---|---|---|---|---|---|
| 37 | 6-(7)Cl | $CH_3$ | 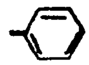 | 138-140 | Waschbenzin | 72 |
| 38 | 6-Cl | $C(CH_3)_3$ | $CH_3$ | 160-161 | Waschbenzin | 57 |
| 39 | 6-Cl | 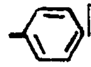 | $CH_3$ | 121-122 | Waschbenzin | 92 |
| 40 | 6-Cl | 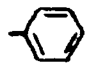 | $C_4H_9$ | 98-100 | Waschbenzin | 89 |
| 41 | 6-Cl | 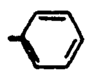 | $CH_2$- 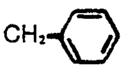 | 168-170 | Essigester/Ligroin | 78 |
| 42 | 6-Cl | 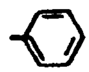 | 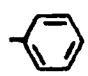 | 150-152 | Waschbenzin | 67 |
| 43 | 6,8-$Cl_2$ | 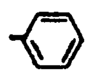 | $CH_3$ | 161-163 | Waschbenzin | 59 |
| 44 | 6,8-$Cl_2$ | 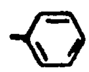 | 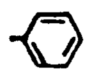 | 180-181 | Waschbenzin | 62 |

Beispiel 11

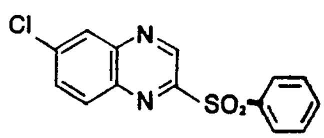

27,3 g (0,1 Mol) 6-Chlor-2-phenylmercapto-chinoxalin werden in 150 ml Essigsäure bei 70-80°C gelöst. Bei dieser Temperatur tropft man 28,3 g (0,25 Mol) 30%iges Wasserstoffperoxid ein. Anschliessend wird das Reaktionsgemisch noch 2 Stunden auf 70-80°C erhitzt und nachfolgend mit 1 Liter Wasser verdünnt. Die abgeschiedenen Kristalle werden abgesaugt, getrocknet und aus Toluol umkristallisiert. Man erhält 24 g (79% d. Th.) 6-Chlor-2-phenylsulfonyl-chinoxalin vom Schmelzpunkt F: 201-202°C.

In entsprechender Weise werden Verbindungen der Formel I

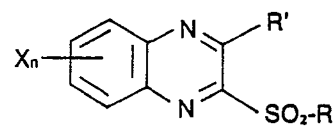

hergestellt.

| Beispiel Nr. | $X_n$ | R' | R | F (°C) | Umkrist. aus | Ausbeute (%) |
|---|---|---|---|---|---|---|
| 13 | 6,8-$Cl_2$ | H | $C_2H_5$ | 121-122 | Waschbenzin | 73 |
| 14 | 6,8-$Cl_2$ | H | i-$C_3H_7$ | 99-100 | Waschbenzin | 65 |
| 15 | 6,8-$Cl_2$ | H | -$CH_2$- 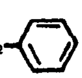 | 127-128 | Waschbenzin | 82 |
| 16 | 6,8-$Cl_2$ | H | -$CH_2$- 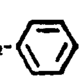 -Cl | 137-138 | Essigester | 69 |

Vorprodukte der Formel II

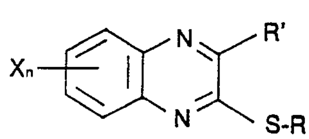

(II)

Methode A

In die Mischung aus 0,1 Mol eines Mercaptans (Thiophenols), R-SH, 100 ml Dioxan und 10 g (0,1 Mol) konz. Natronlauge trägt man bei Raumtemperatur portionsweise 0,1 Mol eines 2-Chlor--chinoxalins ein. Die Mischung wird 1 Stunde bei Raumtemperatur und 4 Stunden bei 50°C nachgerührt, abgekühlt, mit 1 Liter Wasser verdünnt. Das Reaktionsprodukt wird abgesaugt, getrocknet und umkristallisiert.

Auf diesem Wege werden hergestellt:

| Beispiel Nr. | $X_n$ | R' | R | F (°C) | Umkrist. aus |
|---|---|---|---|---|---|
| 11 A | 6-Cl | H | 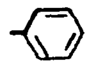 | 73 | Waschbenzin |
| 12 A | 6,8-$Cl_2$ | H | $CH_3$ | 120-122 | Waschbenzin |
| 20 A | 6,8-$Cl_2$ | H | 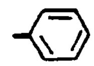 | 130-131 | Waschbenzin |
| 26 A | 5,6,8-$Cl_3$ | H | $CH_3$ | 132-134 | Waschbenzin |
| 27 A | 5,6,8-$Cl_3$ | H | $CH_2$- 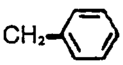 | 138-140 | Waschbenzin |
| 28 A | 5,6,7,8-$Cl_4$ | H | $CH_3$ | 124-126 | Waschbenzin |
| 29 A | 6-$NO_2$ | H | 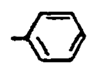 | 104-106 | Waschbenzin |
| 31 A | 6-$CF_3$ | H | $C_4H_9$ | $Kp_{0.08}$: 130-132 | |
| 33 A | 6-$CF_3$ | H | 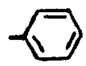 | 69-70 | Waschbenzin |
| 34 A | 6-(7)Cl | $CH_3$ | $CH_3$ | 80-82 | Waschbenzin |
| 35 A | 6-(7)Cl | $CH_3$ | $C_4H_9$ | nicht kristallisiert | |
| 36 A | 6-(7)Cl | $CH_3$ | $CH_2$- 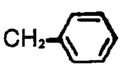 | 64-66 | Waschbenzin |
| 37 A | 6-(7)Cl | $CH_3$ | 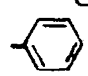 | 92-94 | Waschbenzin |
| 38 A | 6-Cl | $C(CH_3)_3$ | $CH_3$ | nicht kristallisiert | |
| 39 A | 6-Cl | 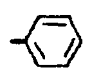 | $CH_3$ | 128-130 | Waschbenzin |
| 40 A | 6-Cl | 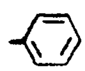 | $C_4H_9$ | 100-102 | Waschbenzin |
| 41 A | 6-Cl | 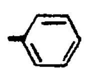 | $CH_2$- 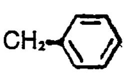 | 100-102 | Waschbenzin |
| 42 A | 6-Cl | 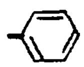 | 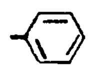 | 110 | Waschbenzin |
| 44 A | 6,8-$Cl_2$ | 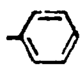 | 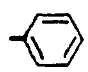 | 162-164 | Waschbenzin |
| 45 A | 6,8-$Cl_2$ | $C(CH_3)_3$ | $CH_3$ | 140-142 | Waschbenzin |

Methode B

0,1 Mol eines 2-Mercapto-chinoxalins werden in 100 ml Äthanol unter Zugabe von 10 g (0,1 Mol) konz. Natronlauge gelöst. Hierzu tropft man bei Raumtemperatur 0,11 Mol eines Alkyl-(Cycloalkyl-, Aralkyl-)halogenids. Das Reaktionsgemisch wird 1 Stunde bei Raumtemperatur und noch 2 Stunden bei 40-50°C nachgerührt, mit Wasser versetzt und abgesaugt. Das gewonnene kristalline Produkt wird getrocknet und umkristallisiert.

Auf diesem Wege werden hergestellt:

| Beispiel Nr. | $X_n$ | R' | R | F (°C) | Umkrist. aus |
|---|---|---|---|---|---|
| 1 B | 6-Cl | H | $CH_3$ | 98 | Waschbenzin |
| 2 B | 6-Cl | H | $C_2H_5$ | 50 | — |
| 3 B | 6-Cl | H | $C_3H_7$ | 39-40 | — |
| 4 B | 6-Cl | H | $C_4H_9$ | $Kp_{0.07}$: 153-155 | |
| 5 B | 6-Cl | H | 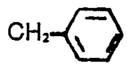 | 78-80 | Waschbenzin |
| 6 B | 6-Cl | H | 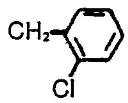 | 89-90 | Waschbenzin |
| 7 B | 6-Cl | H | 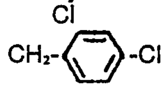 | 95-96 | Waschbenzin |
| 8 B | 6-Cl | H | 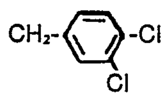 | 107-108 | Waschbenzin |
| 9 B | 6-Cl | H | 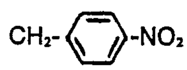 | 129-130 | Tetrachlorkohlenstoff |
| 10 B | 6-Cl | H | 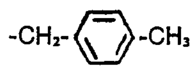 | 66-68 | — |
| 13 B | 6,8-$Cl_2$ | H | $C_2H_5$ | 90 | Waschbenzin |
| 14 B | 6,8-$Cl_2$ | H | i-$C_3H_7$ | 98-100 | Äthanol |
| 15 B | 6,8-$Cl_2$ | H | 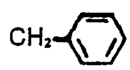 | 87 | Waschbenzin |
| 16 B | 6,8-$Cl_2$ | H | 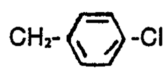 | 97-98 | Toluol |
| 17 B | 6,8-$Cl_2$ | H | 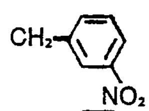 | 124-126 | Waschbenzin |
| 18 B | 6,8-$Cl_2$ | H | 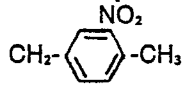 | 102-104 | Waschbenzin |
| 19 B | 6,8-$Cl_2$ | H | $CH_2$-$CH_2$-$N(CH_3)_2$ | 96-98 | Waschbenzin |
| 21 B | 6,7-$Cl_2$ | H | $CH_3$ | 140 | Waschbenzin |
| 22 B | 6,7-$Cl_2$ | H | $C_2H_5$ | 98 | Waschbenzin |
| 23 B | 6,7-$Cl_2$ | H | $C_3H_7$ | 77-78 | Waschbenzin |
| 24 B | 6,7-$Cl_2$ | H | $C_4H_9$ | 60 | Waschbenzin |
| 25 B | 6,7-$Cl_2$ | H | 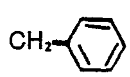 | 114-116 | Waschbenzin |
| 30 B | 6-$CF_3$ | H | $CH_3$ | nicht kristallisiert | |
| 32 B | 6-$CF_3$ | H | 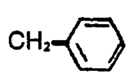 | nicht kristallisiert | |
| 43 B | 6,8-$Cl_2$ | 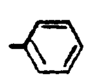 | $CH_3$ | 160-162 | Waschbenzin |

Vorprodukte der Formel IV

1) 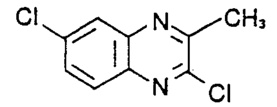

und

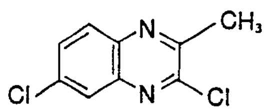

142,5 g (1 Mol) 4-Chlor-1,2-diamino-benzol werden in einer Mischung aus 200 ml Äthanol, 1 Liter Wasser und 200 ml konz. Salzsäure gelöst. Hierzu tropft man bei Raumtemperatur die Lösung von 88 g (1 Mol) Brenztraubensäure in 200 ml Äthanol und rührt anschliessend noch 5 Stunden bei Raumtemperatur nach. Die Mischung wird mit 1 Liter Wasser verdünnt, das unlösliche Produkt abgesaugt, mit Wasser gewaschen und getrocknet. Man erhält in praktisch quantitativer Ausbeute ein Gemisch aus gleichen Mengen 6-Chlor- und 7-Chlor-2-hydroxy-3-methyl-chinoxalin. F: 234-236°C (aus Äthanol)..

194,5 g (1 Mol) 6-(und 7-)Chlor-2-hydroxy-3--methyl-chinoxalin werden in 1,5 Liter Toluol suspendiert. Hierzu gibt man 10 ml Dimethylformamid und leitet dann bei allmählich steigender Temperatur Phosgen ein. Das Einleiten von Phosgen wird noch weitere 5 Stunden fortgesetzt. Dann wird der Überschuss Phosgen durch Einleiten von Stickstoff entfernt. Unlösliche Anteile werden abgesaugt, die Lösung i. Vak. eingedampft. Der verleibende Rückstand wird aus Tetrachlorkohlenstoff umkristallisiert. Ausbeute: 164 g (77% d. Th.) einer 1:1-Mischung aus 2,6- und 2,7-Dichlor-3-methyl-chinoxalin.
F: 104-106°C.

2) 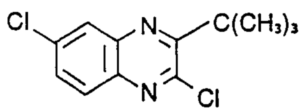

142,5 g (1 Mol) 4-Chlor-1,2-diamino-benzol werden in 1,5 Liter Äthanol mit 144 g (1 Mol) tert.-Butyl-glyoxylsäureester 5 Stunden unter Rückfluss erhitzt. Das abgekühlte Reaktionsgemisch wird mit 3 Liter Wasser verdünnt und abgesaugt. Man löst das Rohprodukt in 2 Liter verdünnter Natronlauge und fällt es nach Filtration unlöslicher Bestandteile durch Ansäuern des Filtrates wieder aus. Nachfolgend wird abgesaugt, mit Wasser gewaschen und aus Äthanol umkristallisiert. Man erhält 207 g (87,6% d. Th.) 6-Chlor--2-hydroxy-3-tert.-butyl-chinoxalin. F. 208°C.

236,7 g (1 Mol) 6-Chlor-2-hydroxy-3-tert.-butyl--chinoxalin werden portionsweise in 1,5 Liter Thionylchlorid eingetragen. Dann tropft man 10 ml Dimethylformamid zu und erhitzt die Mischung allmählich zum Sieden. Nach 5stündigem Kochen wird das überschüssige Thionylchlorid abdestilliert, der Rückstand in 2 Liter Wasser eingerührt. Das ölige Produkt wird mit Toluol ausgeschüttelt, die Toluollösung getrocknet und i. Vak. eingedampft. Als Rückstand verbleiben 242 g (95% d. Th.) des öligen 2,6-Dichlor-3-tert.-butyl-chinoxalins, das in Ligroin glatt löslich und nach GC-Analyse nahezu 100%ig rein ist.

In entsprechender Weise gewinnt man, ausgehend von 3,5-Dichlor-1,2-diamino-benzol das 2,6,8-Trichlor-3-tert.-butyl-chinoxalin der Formel

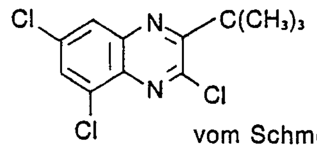
vom Schmelzpunkt F: 104-106°C.

3) 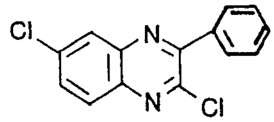

142,5 g (1 Mol) 4-Chlor-1,2-diamino-benzol werden in 1,2 Liter Äthanol mit 165 g (1,1 Mol) Phenylglyoxylsäure 5 Stunden unter Rückfluss erhitzt. Man verdünnt mit 2 Liter Wasser und saugt das Reaktionsprodukt ab. Dieses wird in 2 Liter verd. Natronlauge gelöst und nach Filtration der Lösung durch Ansäuern wieder ausgefällt. Das mit Wasser gewaschene und getrocknete Produkt lässt sich aus Butanol umkristallisieren. Man erhält 160 g (62,3% d. Th.) 6-Chlor-2-hydroxy-3-phenyl-chinoxalin vom Schmelzpunkt F: 202-204°C.

256 g (1 Mol) 6-Chlor-2-hydroxy-3-phenyl-chinoxalin werden wie bei Vorprodukt IV/1 mit Thionylchlorid in Gegenwart von Dimethylformamid umgesetzt. Man erhält 185 g (67% d. Th.) 2,6-Dichlor-3-phenyl-chinoxalin vom F: 106-108°C (Waschbenzin).

In entsprechender Weise erhält man auch 2,6,8-Trichlor-3-phenyl-chinoxalin der Formel

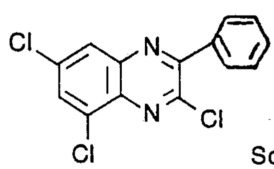
Schmelzpunkt F: 142-144°C.

Die erfindungsgemässen Verbindungen der Formel I weisen starke antimikrobielle, insbesondere antimykotische Wirkungen auf. Sie zeigen ein breites Wirkungsspektrum in vitro, das Dermatophyten, Hefen, Schimmelpilze und biphasische Pilze umfasst. Besonders hervorzuheben ist eine teilweise gute, rasch abtötende Wirkung auf Pilzsporen. Ferner wirken sie gegenüber parasitischen Protozoen insbesondere Trichomonaden, Lamblien, Amoeben und Histomonas-Arten.

Als Indikationsgebiete in der Humanmedizin können beispielsweise genannt werden:

Dermatomykosen und Systemmykosen, die durch Trichophyton mentagrophytes und andere Trichophyton-Arten, Mikrosporon-Arten, Epidermophyton floccosum, Sprosspilze und biphasische Pilze sowie Schimmelpilze hervorgerufen werden, ferner Trichomoniasis, Giardiasis und Amoebiasis. Als Indikationsgebiet in der Tiermedizin können beispielsweise aufgeführt werden:

Alle Dermatomykosen und Systemmykosen, insbesondere solche, die durch die oben genannten Erreger hervorgerufen werden; Infektionen mit Trichomonaden, Lamblien, Hexamites usw.

Ausserdem weisen die erfindungsgemässen Wirkstoffe bei geringer Toxizität und guter Verträglichkeit eine starke antibakterielle Wirksamkeit auf. Diese Eigenschaften ermöglichen ihre Verwendung als Wirkstoffe ausser in der Medizin auch als Stoffe zur Konservierung von anorganischen und organischen Materialien, insbesondere von organischen Materialien aller Art, z. B. Polymeren, Schmiermitteln, Farben, Fasern, Leder, Papier und Holz, von Lebensmitteln, Kosmetika, wie Cremes und Salben sowie von Wasser.

Die erfindungsgemässen Wirkstoffe sind gegen ein sehr breites Spektrum von Mikroorganismen wirksam. Mit ihrer Hilfe können z. B. Gram-negative und Gram-positive Bakterien und bakterienähnliche Mikroorganismen bekämpft sowie die durch diese Erreger hervorgerufenen Erkrankungen verhindert, gebessert und/oder geheilt werden. Sie sind daher besonders gut zur Prophylaxe und Chemotherapie von lokalen und systemischen Infektionen in der Human- und Tiermedizin geeignet, die durch Bakterien und bakterienähnliche Mikroorganismen hervorgerufen werden.

Beispielsweise können lokale und/oder systemische Erkrankungen behandelt und/oder verhindert werden, die durch die folgenden Erreger oder durch Mischungen der folgenden Erreger verursacht werden:

Micrococcaceae, wie Staphylokokken, z. B. Staphylococcus aureus, Staph. epidermidis;

Lactobacteriaceae, wie Streptokokken, z. B. Streptococcus pyogenes, α- bzw. β-hämolysierende Streptokokken, nicht (γ-)hämolysierende Streptokokken, Str. viridans, Str. faecalis (Enterokokken);

Corynebacteriaceae, wie Corynebakterien, z.B. Corynebacterium diphtheriae, C. pyogenes, C. diphtheroides, C. acnes, C. parvum;

Enterobacteriaceae, wie Escherichiae-Bakterien der Coli-Gruppe: z. B. Escherichia coli, Klebsiella-Bakterien, z. B. K. pneumoniae, Proteae-Bakterien der Proteus-Gruppe: z. B. Proteus vulgaris, Pr. mirabilis, Pseudomonadaceae, wie Pseudomonas-Bakterien, z. B. Pseudomonas aeruginosa.

Die obige Aufzählung von Erregern ist lediglich beispielhaft und keineswegs beschränkend aufzufassen.

Als Krankheiten, die durch die erfindungsgemässen Wirkstoffe verhindert, gebessert und/ oder geheilt werden können, seien beispielsweise genannt:

Erkrankungen der Atmungswege und des Rachenraumes;

Otitis; Pharyngitis; Pyelonephritis; Cystitis; sowie lokale Infektionen z. B. der Haut und lokal zugänglicher Schleimhäute.

Zur Erfindung gehören pharmazeutische Zubereitungen, die neben nicht toxischen, inerten pharmazeutisch geeigneten Trägerstoffen einen oder mehrere erfindungsgemässe Wirkstoffe enthalten oder die aus einem oder mehreren erfindungsgemässen Wirkstoffen bestehen sowie Verfahren zur Herstellung dieser Zubereitungen.

Unter nicht toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Lösungen, Suspensionen und Emulsionen, Pasten, Salben, Gele, Cremes, Lotions, Puder und Sprays genannt.

Der oder die Wirkstoffe können gegebenenfalls mit einem oder mehreren der oben angegebenen Trägerstoffen auch in mikroverkapselter Form vorliegen.

Salben, Pasten, Cremes und Gele können neben dem oder den Wirkstoffen die üblichen Trägerstoffe enthalten, z. B. tierische und pflanzliche Fette, Wachse, Paraffine, Stärke, Tragant, Cellulosederivate, Polytähylenglykole, Silicone, Bentonite, Kieselsäure, Talkum und Zinkoxid oder Gemische dieser Stoffe.

Puder und Sprays können neben dem oder den Wirkstoffen die üblichen Trägerstoffe enthalten, z.B. Milchzucker, Talkum, Kieselsäure, Aluminiumhydroxid, Calciumsilikat und Polyamidpulver oder Gemische dieser Stoffe. Sprays können zusätzlich die üblichen Treibmittel z. B. Chlorfluorkohlenwasserstoffe enthalten.

Lösungen und Emulsionen können neben dem oder den Wirkstoffen die üblichen Trägerstoffe wie Lösungsmittel, Lösungsvermittler und Emulgatoren, z. B. Wasser, Äthylacetat, Benzylalkohol, Propylenglykol, 1,3-Butylenglykol, Dimethylformamid, Öle, insbesondere Baumwollsaatöl, Erdnussöl, Maiskeimöl, Olivenöl, Ricinusöl und Sesamöl, Glycerin, Glycerinformal, Tetrahydrofurfurylalkohol, Polyäthylenglykole und Fettsäureester des Sorbitans oder Gemische dieser Stoffe enthalten.

Suspensionen können neben dem oder den Wirkstoffen die üblichen Trägerstoffe wie flüssige Verdünnungsmittel, z. B. Wasser, Äthylalkohol, Propylenglykol, Suspendiermittel, z. B. äthoxylierte Isostearylalkohole, Polyoxyäthylensorbit- und Sorbitanester, mikrokristalline Cellulose, Aluminiummetahydroxid, Betonit, Agar-Agar und Tragant oder Gemische dieser Stoffe enthalten.

Die oben aufgeführten pharmazeutischen Zubereitungen können ausser den erfindungsgemässen Wirkstoffen auch weitere pharmazeutische Wirkstoffe enthalten.

Die Herstellung der oben aufgeführten pharmazeutischen Zubereitungen erfolgt in üblicher Weise nach bekannten Methoden, z. B. durch Mischen des oder der Wirkstoffe mit dem oder den Trägerstoffen.

Für die Anwendung im technischen Bereich bzw. auf dem Hygienesektor können die erfindungsgemässen Wirkstoffe in nicht modifizierter Form oder mit einem Träger, z. B. dispersiert auf einem feinverteilten Feststoff, oder als Staub eingesetzt werden. Solche Gemische können auch in Wasser unter Zuhilfenahme eines Netzmittels dispersiert werden und die resultierenden Emulsionen können als Spray verwendet werden. Bei anderen Arbeitsweisen können die Produkte als Wirkstoffe in Lösungsmittel-Lösungen, Öl-in-Wasser- oder Wasser-in-Öl-Emulsionen verwendet werden. Die Mischungen können als Konzentrate formuliert werden und danach mit weiteren flüssigen oder festen Hilfsstoffen verdünnt werden, um die Endbehandlungsmischung herzustellen. Gute Ergebnisse werden insbesondere dann erhalten, wenn Mischungen verwendet werden, die 0,5 bis 3% Wirkstoff enthalten.

Beispiel A
Antimykotische In-vitro-Wirksamkeit
Versuchsbeschreibung:

Die minimalen Hemmkonzentrationen (MHK) gegen Pilze wurden im Reihenverdünnungstest mit Keiminokula von durchschnittlich $5\times10^4$ Keimen/ml Substrat ermittelt. Als Nährmedium dienten

a) für Dermatophyten und Schimmelpilze
Sabourad's milieu d'épreuve
b) für Hefen:
Fleischextrakt-Traubenzucker-Bouillon.

Die Bebrütungstemperatur betrug 28°C, die Bebrütungsdauer lag bei 24 bis 96 Stunden.

Die erfindungsgemässen Verbindungen zeigten vor allem gegenüber Trichophyton mentagrophytes, Microsponum canis, Candida albicans, Torulopsis glabrata und Aspergillus fumigatus günstige MHK-Werte.

Beispiel B
Antibakterielle In-Vitro-Wirksamkeit
Versuchsbeschreibung:

Die minimalen Hemmkonzentrationen gegen Bakterien wurden im Agarverdünnungstest ermittelt. Dazu wurden verschiedene Präparatkonzentrationen zusammen mit dem Teststamm in flüssigem DST-Agar-Medium suspendiert und in Petrischalen (Durchmesser 5 cm) gegossen. Die Keimeinsaat pro Platte betrug $5\times10^3$ Keime. Als MHK wird die geringste Präparatkonzentration bezeichnet, bei der innerhalb von 24 Stunden keine Koloniebildung mehr stattfand.

Die erfindungsgemässen Verbindungen zeigten vor allem gegenüber Streptococcus pyogenes W., Staphylococcus aureus 133. Klebsiella 8085 und Proteus vulgaris 1017 günstige MHK-Werte.

Beispiel C
Trichomonazide Wirksamkeit

Die Prüfung in vitro erfolgte im Reihenverdünnungstest an einem Trichomonas vaginalis-Stamm, der durch Züchtung auf Antibiotica-haltigen Nährböden weitgehend steril gehalten wurde. Verwendet wurde 2-3 Tage altes Kultur-Material.

Für die Versuche wurden mit Trichomonas-Agar beschichtete Schrägröhrchen mit 4 ml einer Nährlösung überschichtet und mit 0,5 ml der entsprechend verdünnten Prüfsubstanz beschickt. Anschliessend wurde jedes Kulturröhrchen mit ca. $10^6$ Trichomonaden in 0,5 ml Nährlösung infiziert. Nach einer Bebrütungszeit von 48 Stunden bei 34°C wurde Kulturmaterial zur mikroskopischen Untersuchung entnommen. Die Beurteilung der Versuche erfolgte durch Vergleich der Ergebnisse der behandelten Kulturen mit denen der unbehandelten Kontrollen. Bei bestimmten minimalen Hemmkonzentrationen waren keine lebenden Trichomonaden mehr nachweisbar.

Zur Prüfung in vivo auf trichomonazide Wirksamkeit wurden weisse Mäuse (z. B. $CF_1$ Winkelmann) mit Kulturmaterial von Trichomonas foetus oder T. vaginalis (1-15 Millionen Trichomonaden/Maus) intraperitoneal infiziert. Die im Wasser gelösten oder suspendierten Wirkstoffe wurden oral mit der Schlundsonde verabreicht und zwar 4 Stunden vor der Infektion und weitere 4 mal jeweils im Abstand von 24 Stunden. Am 7. Tag p. inf. wurden die Mäuse getötet und seziert und die makroskopisch nachweisbaren Abszesse bzw. das vermehrte Peritoneal-Exsudat mikroskopisch auf Parasiten untersucht. Zur Auswertung dieser Versuche wurden die makroskopischen Befunde sowie die Zahl der Tiere mit positivem mikroskopischem Erregernachweis in den einzelnen Versuchsgruppen mit den Ergebnissen der Kontrollgruppe verglichen.

Unter den genannten Versuchsbedingungen erwiesen sich die erfindungsgemässen Verbindungen als voll wirksam in Konzentrationen von 1000 bis 10 mcg/ml in vitro und 5000 bis 10 mg/kg/d im Tierversuch.

Die erfindungsgemässen Wirkstoffe zeigen vor allem gegenüber Trichomonas vaginalis und Trichomonas foetus gute Wirksamkeit.

**Patentansprüche**

1. 2-Sulfonyl-chinoxaline der Formel I

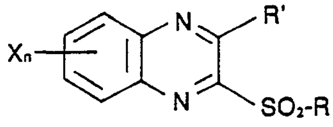

(I)

in der

X ein Halogenatom, Nitro oder Trifluormethyl,

R' ein Wasserstoffatom, einen Alkylrest mit 1 bis 4 Kohlenstoffatomen, einen Cycloalkylrest mit 5 oder 6 Kohlenstoffatomen oder einen Phenylrest, der weiterhin substituiert sein kann durch Halogen, Nitro oder Trifluormethyl,

R einen Alkylrest mit 1 bis 6 Kohlenstoffatomen, der durch Halogen, einen Alkoxyrest mit

bis zu 4-C-Atomen, eine Amino- oder Dimethylaminogruppe substituiert sein kann, einen Cycloalkylrest mit 5 oder 6 Kohlenstoffatomen, einen Phenylalkylrest mit 1 bis 3 Kohlenstoffatomen im Alkylteil, dessen Phenylrest weiterhin durch Halogen, Nitro, Methyl, Methoxy oder Trifluormethyl substituiert sein kann oder einen Phenylrest, der durch Alkyl mit 1-4 C-Atomen, Trifluormethyl, Alkoxy mit 1-4 C-Atomen oder Halogen substituiert sein kann, und

n 1-4

bedeuten.

2. Verfahren zur Herstellung von 2-Sulfonyl--chinoxalinen der Formel I

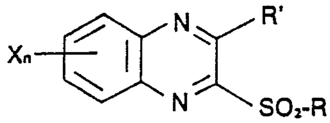

(I)

in der

X ein Halogenatom, Nitro oder Trifluormethyl,

R' ein Wasserstoffatom, einen Alkylrest mit 1 bis 4 Kohlenstoffatomen, einen Cycloalkylrest mit 5 oder 6 Kohlenstoffatomen oder einen Phenylrest, der weiterhin substituiert sein kann durch Halogen, Nitro oder Trifluormethyl,

R einen Alkylrest mit 1 bis 6 Kohlenstoffatomen, der durch Halogen, einen Alkoxyrest mit bis zu 4 C-Atomen, eine Aminogruppe oder Dimethylaminogruppe substituiert sein kann, einen Cycloalkylrest mit 5 oder 6 Kohlenstoffatomen, einen Phenylalkylrest mit 1 bis 3 Kohlenstoffatomen im Alkylteil, dessen Phenylrest weiterhin durch Halogen, Nitro, Methyl, Methoxy oder Trifluormethyl substituiert sein kann oder einen Phenylrest, der durch Alkyl mit 1-4 C-Atomen, Trifluormethyl, Alkoxy mit 1-4 C-Atomen oder Halogen substituiert sein kann, und

n 1-4

bedeuten, dadurch gekennzeichnet, dass man 2-Sulfenyl-chinoxaline der Formel II

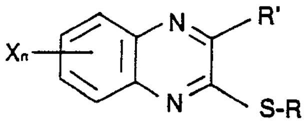

(II)

in der X, R', R und n die oben genannte Bedeutung haben, in an sich bekannter Weise entweder

a) mit Wasserstoffperoxid in organischen Lösungsmitteln bei 20-120°C, vorzugsweise bei 50 bis 90°C, oder

b) mit elementarem Chlor in Gegenwart von Wasser bei höchstens +10°C, vorzugsweise bei 0-5°C, oder

c) mit Permanganat in einem Verdünnungsmittel bei 0-100°C, vorzugsweise bei 10-70°C umsetzt.

3. Arzneimittel, gekennzeichnet durch einen Gehalt an mindestens einem 2-Sulfonyl-chinoxalin gemäss Anspruch 1.

4. Verfahren zur Herstellung eines Arzneimittels, dadurch gekennzeichnet, dass man 2-Sulfonyl-chinoxaline gemäss der Formel in Anspruch 1 mit inerten, nichttoxischen, pharmazeutisch geeigneten Trägerstoffen vermischt.

**Claims**

1. 2-Sulphonyl-quinoxalines of the formula I

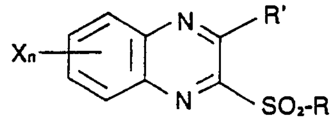

(I)

in which

X denotes a halogen atom, nitro or trifluoromethyl,

R' denotes a hydrogen atom, an alkyl radical with 1 to 4 carbon atoms, a cycloalkyl radical with 5 or 6 carbon atoms or a phenyl radical which can also be substituted by halogen, nitro or trifluoromethyl,

R denotes an alkyl radical with 1 to 6 carbon atoms which can be substituted by halogen, an alkoxy radical with up to 4 C atoms, or an amino or dimethylamino group, a cycloalkyl radical with 5 or 6 carbon atoms, a phenylalkyl radical with 1 to 3 carbon atoms in the alkyl part, the phenyl radical of which can also be substituted by halogen, nitro, methyl, methoxy or trifluormethyl or a phenyl radical which can be substituted by alkyl with 1-4 C atoms, trifluoromethyl, alkoxy with 1-4 C atoms or halogen, and

n denotes 1-4.

2. Process for the preparation of 2-sulphonyl--quinoxalines of the formula I

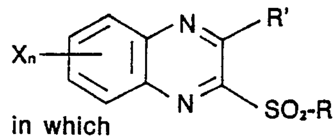

(I)

in which

X denotes a halogen atom, nitro or trifluoromethyl,

R' denotes a hydrogen atom, an alkyl radical with 1 to 4 carbon atoms, a cycloalkyl radical with 5 or 6 carbon atoms or a phenyl radical which can also be substituted by halogen, nitro or trifluoromethyl,

R denotes an alkyl radical with 1 to 6 carbon atoms which can be substituted by halogen, an alkoxy radical with up to 4 C atoms, or an amino or dimethylamino group, a cycloalkyl radical with 5 or 6 carbon atoms, a phenylalkyl radical with 1 to 3 carbon atoms in the alkyl part, the phenyl radical of which can also be substituted by halogen, nitro, methyl, methoxy or trifluoromethyl or a phenyl radical which can be substituted by alkyl with 1-4 C atoms, trifluoromethyl, alkoxy with 1-4 C atoms or halogen, and

n denotes 1-4,

characterised in that 2-sulphenyl-quinoxalines of the formula (II)

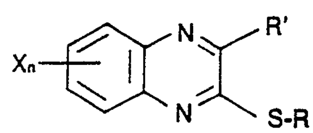

(II)

in which

X, R', R and n have the meaning indicated above, are reacted in a manner known per se either

a) with hydrogen peroxide in organic solvents at 20-120°C, preferably at 50-90°C, or

b) with elementary chlorine in the presence of water at a maximum of +10°C, preferably at 0-5°C, or

c) with permanganate in a diluent at 0-100°C, preferably at 10-70°C.

3. Medicaments, characterised in that they contain at least one 2-sulphonyl-quinoxaline according to Claim 1.

4. Process for the preparation of a medicament, characterised in that 2-sulphonyl-quinoxalines according to the formula in Claim 1 are mixed with inert, non-toxic pharmaceutically suitable excipients.

**Revendications**

1. 2-sulfonyl-quinoxalines de formule I:

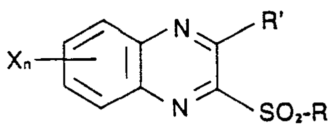

(I)

dans laquelle:

X est un atome d'halogène, un groupe nitro ou trifluorométhyle,

R' est un atome d'hydrogène, un reste alkyle ayant 1 à 4 atomes de carbone, un reste cycloalkyle ayant 5 ou 6 atomes de carbone ou un reste phényle qui peut en outre être substitué par un halogène ou par un groupe nitro ou trifluorométhyle,

R est un reste alkyle ayant 1 à 6 atomes de carbone, qui peut être substitué par un halogène, par un reste alkoxy ayant jusqu'à 4 atomes de carbone, par un groupe amino ou par un groupe diméthylamino, un reste cycloalkyle ayant 5 ou 6 atomes de carbone, un reste phénylalkyle ayant 1 à 3 atomes de carbone dans la partie alkyle, dont le reste phényle peut en outre être substitué par un halogène ou par un groupe nitro, méthyle, méthoxy ou trifluorométhyle, ou un reste phényle qui peut être substitué par un radical alkyle ayant 1 à 4 atomes de carbone, trifluorométhyle, alkoxy ayant 1 à 4 atomes de carbone ou un halogène, et

n est ègal à 1-4.

2. Procédé de production de 2-sufonyl-quinoxalines de formule I:

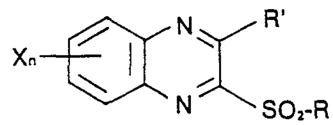

(I)

dans laquelle:

X est un atome d'halogène, un groupe nitro ou trifluorométhyle,

R' est un atome d'hydrogène, un reste alkyle ayant 1 à 4 atomes de carbone, un reste cycloalkyle ayant 5 ou 6 atomes de carbone ou un reste phényle qui peut en outre être substitué par un halogène ou par un groupe nitro ou trifluorométhyle,

R est un reste alkyle ayant 1 à 6 atomes de carbone, qui peut être substitué par un halogène, par un reste alkoxy ayant jusqu'à 4 atomes de carbone, par un groupe amino ou par un groupe diméthylamino, un reste cycloalkyle ayant 5 ou 6 atomes de carbone, un reste phénylalkyle ayant 1 à 3 atomes de carbone dans la partie alkyle, dont le reste phényle peut en outre être substitué par un halogène ou par un groupe nitro, méthyle, méthoxy ou trifluorométhyle, ou un reste phényle qui peut être substitué par un radical alkyle ayant 1 à 4 atomes de carbone, trifluorométhyle, alkoxy ayant 1 à 4 atomes de carbone ou un halogène, et

n est égal à 1-4,

caractérisé en ce qu'on fait réagir des 2-sulfényl-quinoxalines de formule II:

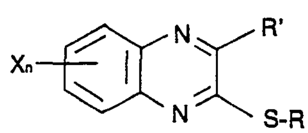

(II)

dans laquelle X, R', R et n on la définition donnée ci-dessus, d'une manière connue

a) avec du peroxyde d'hydrogène dans des solvants organiques à 20-120°C, de préférence à 50-90°C, ou

b) avec du chlore élémentaire en présence d'eau à une température maximale de +10°C, de préférence à 0-5°C, ou

c) avec du permanganate dans un diluant à 0-100°C, de préférence à 10-70°C.

3. Médicament, caractérisé par une teneur en au moins une 2-sulfonyl-quinoxaline suivant la revendication 1.

4. Procédé de production d'un médicament, caractérisé en ce qu'on mélange des 2-sulfonyl--quinoxalines répondant à la formule suivant la revendication 1, avec des supports inertes non toxiques pharmaceutiquement acceptables.